(19)

**Europäisches Patentamt**
**European Patent Office**
**Office européen des brevets**

(11)  **EP 4 613 300 A1**

(12)  **EUROPEAN PATENT APPLICATION**

(43) Date of publication:
**10.09.2025  Bulletin 2025/37**

(51) International Patent Classification (IPC):
***A61M 5/315*** (2006.01)

(21) Application number: 24162403.0

(22) Date of filing: **08.03.2024**

(52) Cooperative Patent Classification (CPC):
**A61M 5/31513; A61M 5/31511; A61M 5/31515**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(71) Applicant: **Becton, Dickinson and Company**
**Franklin Lakes, NJ 07417-1880 (US)**

(72) Inventors:
- **FLIPPE, Marc**
  **38640 CLAIX (FR)**
- **RODRIQUEZ SAN JUAN, Nestor**
  **Hamburg, NJ 07419 (US)**
- **VAXELAIRE, Jérémie**
  **74100 Ambilly (FR)**
- **PERRIN, Eloïse**
  **38320 Eybens (FR)**

(74) Representative: **Germain Maureau**
**12, rue Boileau**
**69006 Lyon (FR)**

(54)  **LOW LUBRICATION SYRINGE PLUNGER STOPPER WITH IMPROVED SYRINGE BARREL SURFACE CONTACT**

(57)  Provided herein is a syringe including a syringe barrel and a plunger assembly axially movable within a chamber of the syringe barrel. The plunger assembly includes a plunger rod having a threaded extension member at a distal end thereof and a stopper secured to the extension member. The stopper includes a main body portion that defines a threaded inner cavity at a proximal end thereof that mates with the threaded extension member, and a plurality of ribs extending around an outer circumference of the main body portion, with at least one of the plurality of ribs positioned on the main body portion so as to be axially aligned with the threaded inner cavity. An interference is present between the threaded extension member and the threaded inner cavity when mated together that applies a radially outward pressure compensation to the rib that is axially aligned with the threaded inner cavity.

**EP 4 613 300 A1**

**Description**

**BACKGROUND OF THE INVENTION**

Field of the Invention

The present disclosure relates generally to a plunger assembly for use with a syringe and, more particularly, to a plunger assembly having a low lubrication stopper that mates with a plunger rod so as to have an interference between a cavity in the stopper and the plunger rod.

Description of Related Art

Medical injection devices, such as syringes, are used in a variety of environments for administering liquids (e.g., medications or drugs) to a patient. Many syringes are provided as prefilled syringes, which provide the convenience of rapidly delivering the liquid therein to a patient without the need to first aspirate the medication from another container and meter its volume. For dispensing fluids, the prefilled syringe will typically include a syringe barrel with a plunger assembly inserted through an open proximal end of the barrel and an opening at the opposite distal end adapted to receive a needle therein by which a fluid is injected into the patient. The plunger assembly typically includes an elongated plunger rod extending out of the barrel, and a plunger head or stopper disposed at the distal end of the plunger rod. The stopper is typically made of elastomeric material and is adapted to ensure the container closure integrity (CCI) of a syringe when the stopper is inserted into the syringe.

Existing stopper designs include a stopper body having a tail portion disposed at its proximal end adapted for attachment to the distal end of the plunger rod, and a head portion disposed at its distal end adapted to interfit with the barrel of the syringe. The tail portion of the stopper may include a cavity formed therein configured to receive a distal head of the plunger rod, so as to engage the plunger rod with the stopper. According to some embodiments, each of the cavity and the plunger head may be configured to provide a threaded engagement therebetween to secure the plunger rod to the stopper. The head portion of the stopper may include a plurality of annular, outwardly protruding ribs formed on an external cylindrical wall thereof, with the ribs applying a contact pressure against an inner surface of the syringe barrel to form a seal therewith that ensures the CCI of the syringe. A series of two or three ribs, for example, may be spaced apart longitudinally along the stopper main body, with one or more of the ribs positioned back near the tail portion, such that those rib(s) are formed on the stopper at a location that is axially/longitudinally aligned with where the cavity is formed in the stopper.

In many existing syringes, it is recognized that it is desirable to provide a lubricant on an outer surface of the stopper, so as to reduce the friction between the stopper and inner surface of the syringe barrel as the stopper is moved through the syringe barrel (i.e., the gliding force). More recently, however, there has been a shift towards the stopper being configured as a lubricant-free or low lubricant stopper, due to some stopper lubricants including per- and poly- fluoroalkyl substances (PFAS) that might leach into the medicament or other solution contained in the syringe. While such lubricant-free or low lubricant stoppers may reduce/eliminate the issue of PFAS, it is recognized that these stoppers are highly sensitive to contact pressure (due to an increased gliding force), with it recognized that failing to maintain a consistent contact pressure can negatively impact the ability to maintain CCI in the syringe.

In many plunger assemblies, the ability to maintain a consistent contact pressure between the stopper and the syringe barrel is complicated by the design of the stopper and its mating to the plunger rod. Specifically, for the more proximal ribs that are positioned on the stopper at a location where the cavity is formed in the stopper, a lower amount of stopper material is present that can provide a radially outward-directed contact force to the rib(s). Insertion of the plunger rod into the inner cavity does not address this reduction in the amount of stopper material, as there is typically at least a minimal amount of radial play (i.e., a gap/separation) between the plunger rod and the cavity after the plunger rod has been threaded into the cavity.

Accordingly, a need exists in the art for a plunger assembly design that is able to maintain a consistent radial contact pressure between the stopper and the syringe barrel, especially for lubricant-free or low lubricant stoppers. The plunger assembly would be able to maintain a consistent contact pressure for all ribs on the stopper, including those ribs axially aligned with the inner cavity.

**SUMMARY OF THE INVENTION**

Provided herein is a syringe including a syringe barrel having a barrel proximal end and a barrel distal end and defining a chamber configured for containing a fluid therein, and a plunger assembly axially movable within the chamber of the syringe barrel between a retracted position and an advanced position. The plunger assembly includes a plunger rod having a plunger rod proximal end and a plunger rod distal end, with a threaded plunger head formed at the plunger rod distal end. The plunger assembly also includes a stopper secured to the plunger rod distal end. The stopper includes a main body portion defining a proximal end and a distal end, with the main body defining a threaded inner cavity at the proximal end configured to mate with the threaded plunger head to secure the stopper to the plunger rod. The stopper also includes a plurality of ribs extending from an outer surface of the main body portion and around an outer circumference of the main body portion, with at least one of the plurality of ribs positioned on the main body portion so as to be axially aligned with the threaded inner cavity. With the threaded plunger head mated with the threaded inner cavity, an interference is present between the threaded plunger head and the threaded inner cavity that applies a radially outward pressure compensation to the at least one rib that is axially aligned with the threaded inner cavity.

In certain configurations, the plurality of ribs comprises a first rib and a second rib, and wherein the second rib is axially aligned with the portion of the threaded inner cavity.

In certain configurations, the plurality of ribs further comprises a third rib, and wherein each of the second rib and the third rib are axially aligned with portions of the threaded inner cavity.

In certain configurations, each of the threaded extension member and the threaded inner cavity comprises a thread, the thread configured as a cylindrical thread or a conical thread.

In certain configurations, the interference between the threads of the threaded extension member and the threaded inner cavity is between 0.5% and 15%, as defined by:

$$\text{Interference} = (\text{abs}\,(\text{ID\_stopper thread} - \text{OD\_plunger rod thread}) \,/\, \text{OD\_plunger rod thread})$$

where ID_stopper thread is an inner diameter of the stopper cavity thread and OD_plunger rod thread is an outer diameter of the plunger rod thread.

In certain configurations, the interference is determined by a pitch of the inner cavity thread and a pitch of the extension member thread.

In certain configurations, the interference is determined by an outer diameter of the extension member thread and an inner diameter of the inner cavity thread.

In certain configurations, the thread of each of the threaded extension member and the threaded inner cavity comprises a crest, and wherein the crest of the threaded extension member or the threaded inner cavity is in interference with the other of the threaded extension member or the threaded inner cavity.

In certain configurations, the radially outward pressure compensation applied to the at least one rib by the interference between the threaded extension member and the threaded inner cavity is from 10 to 50%.

In certain configurations, another interference is present between the plurality of ribs and an inner surface of the syringe barrel.

In certain configurations, the another interference between the plurality of ribs and the inner surface of the syringe barrel is between 2% and 10%%, as determined by: Interference = (($OD_{ribs}$ /$ID_{barrel}$) - 1) x 100, wherein $OD_{ribs}$ is an outer diameter of the ribs and $ID_{barrel}$ is an inner diameter of the syringe barrel.

In certain configurations, the plurality of ribs apply a contact pressure against the inner surface of the syringe barrel of between 1 and 60 MPa.

In certain configurations, the stopper comprises a lubricant-free or low lubricant stopper.

In certain configurations, the stopper comprises one or more layers of inert material formed on the plurality of ribs and the outer surface of the main body portion.

In certain configurations, the inert material formed comprises one or more of a fluoropolymer, an ultra-high-molecular-weight polyethylene (UHMWPE), or a thermoplastic elastomer (TPE) free of per- and poly- fluoroalkyl substances (PFAS).

**BRIEF DESCRIPTION OF THE DRAWINGS**

FIG. 1 is a perspective view of a syringe, with which embodiments of the disclosure may be implemented;
FIG. 2 is an exploded view of the syringe of FIG. 1;
FIG. 3 is a side view of the stopper included in the syringe of FIG. 1, according to a non-limiting embodiment described herein;
FIG. 4 is a side view of the extension member of the plunger rod included in the syringe of FIG. 1, according to a non-limiting embodiment described herein;
FIG. 5 is a side cross-sectional view of the mated stopper and extension member of FIG. 1, taken along line 5-5;
FIG. 6 is a detail view of area I of FIG. 5;
FIG. 7 is a detail view of area II of FIG. 5; and
FIG. 8 is a side view of a stopper useable in the syringe of FIG. 1, according to a non-limiting embodiment described herein.

## DESCRIPTION OF THE INVENTION

The following description is provided to enable those skilled in the art to make and use the described embodiments contemplated for carrying out the invention. Various modifications, equivalents, variations, and alternatives, however, will remain readily apparent to those skilled in the art. Any and all such modifications, variations, equivalents, and alternatives are intended to fall within the spirit and scope of the present invention.

For purposes of the description hereinafter, the terms "upper", "lower", "right", "left", "vertical", "horizontal", "top", "bottom", "lateral", "longitudinal", and derivatives thereof shall relate to the invention as it is oriented in the drawing figures. However, it is to be understood that the invention may assume various alternative variations, except where expressly specified to the contrary. It is also to be understood that the specific devices illustrated in the attached drawings, and described in the following specification, are simply exemplary embodiments of the invention. Hence, specific dimensions and other physical characteristics related to the embodiments disclosed herein are not to be considered as limiting.

In the present disclosure, the distal end of a component or of a device means the end furthest away from the hand of the user and the proximal end means the end closest to the hand of the user, when the component or device is in the use position. Thus, with a syringe for example, the distal end is the end thereof that includes a needle (or luer connection), while the proximal end is where the user engages the plunger (i.e., the plunger thumb press). Similarly, in this application, the terms "in the distal direction" and "distally" mean in the direction toward the distal tip of the syringe, and the terms "in the proximal direction" and "proximally" mean in the direction opposite the direction of the distal tip of the syringe (i.e., in the direction of the plunger thumb press).

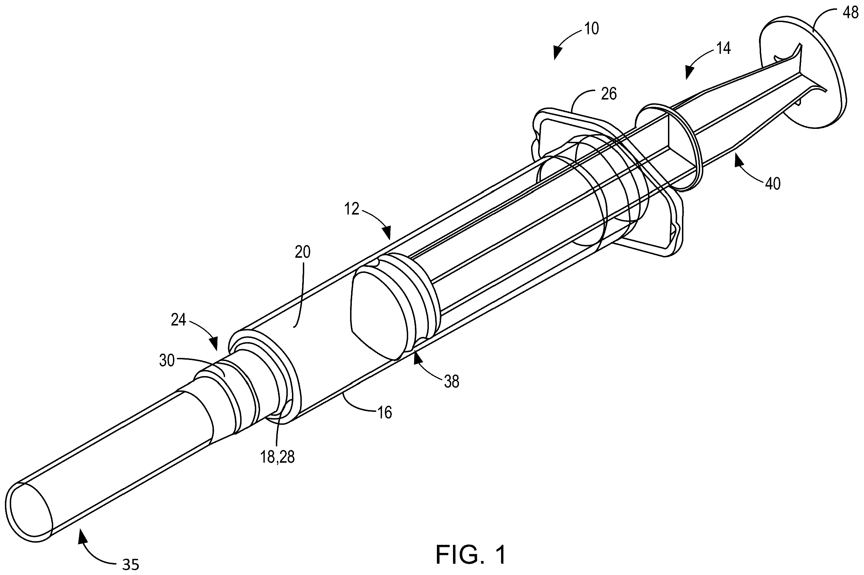

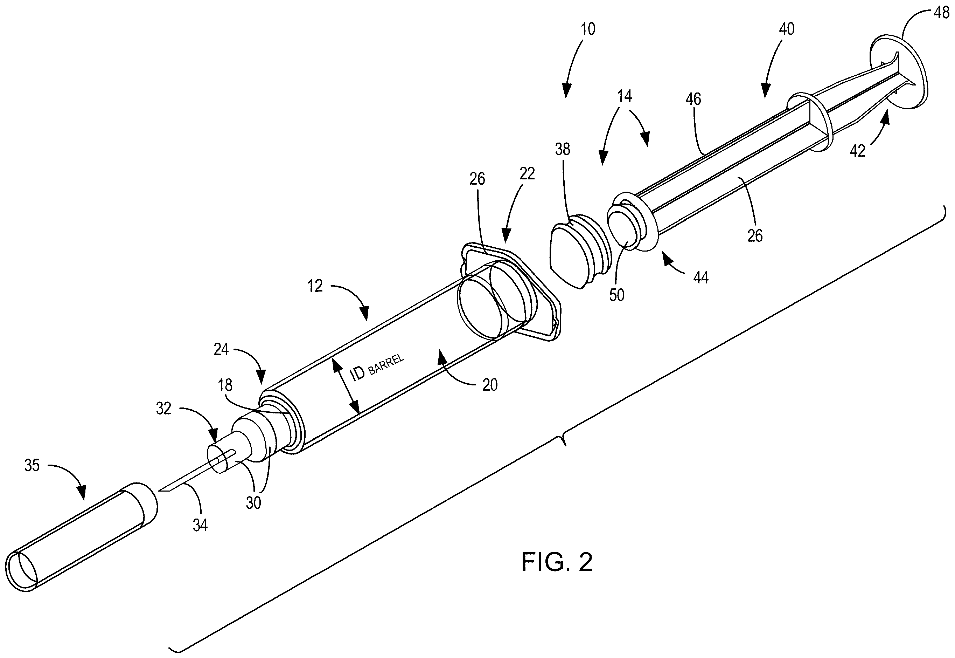

Referring to FIGS. 1 and 2, shown is a non-limiting embodiment of a syringe 10 with which aspects or embodiments of the disclosure may be implemented. According to some aspects of the disclosure, the syringe 10 may be provided as a prefilled syringe, which provides the convenience of rapidly delivering the liquid therein to a patient without the need to first aspirate the medication from another container and meter its volume.

As shown in FIGS. 1 and 2, the syringe 10 generally includes a syringe barrel 12 and a plunger assembly 14. The plunger assembly 14 is movable within the syringe barrel 12 along a longitudinal axis to an advanced position to facilitate administering of an injectable fluid (e.g., medication) to a patient, for example. The syringe barrel 12 is formed of a generally cylindrical wall 16 and an end member 18 that collectively define a chamber 20 for retaining fluid therein. The syringe barrel 12 includes an open proximal end 22 configured to receive the plunger assembly 14 therein and a distal end 24 at which end member 18 is positioned. The proximal end 22 of the syringe barrel 12 may include a flange 26 to facilitate handling and positioning of the syringe 10 and to maintain the relative position of the syringe barrel 12 with respect to the plunger assembly 14 during medication administration. At the distal end 24, the end member 18 may include a shoulder 28 which narrows with respect to the cylindrical outer wall 16, as well as a hub portion 30 extending out distally from shoulder 28. The hub portion 30 is formed as a partially hollow member that defines a channel 32 therethrough in fluid communication with the chamber 20. A needle 34 is attached to the hub portion 30 within channel 32, such as by being glued or otherwise secured to the hub portion 30. According to some aspects of the disclosure, syringe 10 may further include a cover 35 that couples to the hub portion 30 of syringe barrel 12 to protect the needle 34.

The plunger assembly 14 of syringe 10 is formed of an elongate plunger rod 36 and a plunger head or stopper 38. The plunger rod 36 may include a main body 40 extending between a plunger proximal end 42 and a plunger distal end 44. In some embodiments, the main body 40 may include a plurality of elongate vanes or walls 46 extending axially along a length thereof between the plunger proximal end 42 and the plunger distal end 44. A thumb press 48 is positioned at the plunger proximal end 42 that may be engaged by a thumb (or other finger) of the user to apply a distally directed force to the plunger assembly 14 to move the plunger rod 36 with respect to the syringe barrel 12. The plunger distal end 44 includes a threaded extension member 50 that extends out and is configured to mate with the stopper 38. The threaded extension member 50 includes a thread 51 thereon that, according to embodiments, may be configured as a cylindrical thread or a conical thread, as non-limiting examples.

The stopper 38 of plunger assembly 14 is positioned at the plunger distal end 44 so as to be movable along with the plunger rod 36 within the chamber 20 of syringe barrel 12. The stopper 38 may be made from a material that is different from the material of the plunger rod 36 and that is capable of forming a tight seal with the syringe barrel 12 as it is advanced therethrough. In some embodiments, the stopper 38 is formed of an elastomeric material such as butyl, styrene butadiene, isoprene, as non-limiting examples. In other embodiments, the stopper 38 may instead be formed of a polymeric material, such as cross-linked or thermoplastic elastomers, as non-limiting examples. According to aspects of the disclosure, whether the stopper 38 is formed of an elastomeric material or a polymeric material, the stopper is formed to be free or substantially free of per- and poly- fluoroalkyl substances (PFAS) - generally, a "PFAS-free" material - that might leach into the medicament or other solution stored in pre-filled syringe 10. As defined herein, the stopper 38 being "free or substantially free of PFAS" is understood to refer to materials that are entirely free of PFAS and/or materials that contain trace amounts of PFAS therein, such as between 0 and 200 parts-per-million (PPM) of PFAS therein and preferably between 0 and 50 PPM of PFAS, as non-limiting examples.

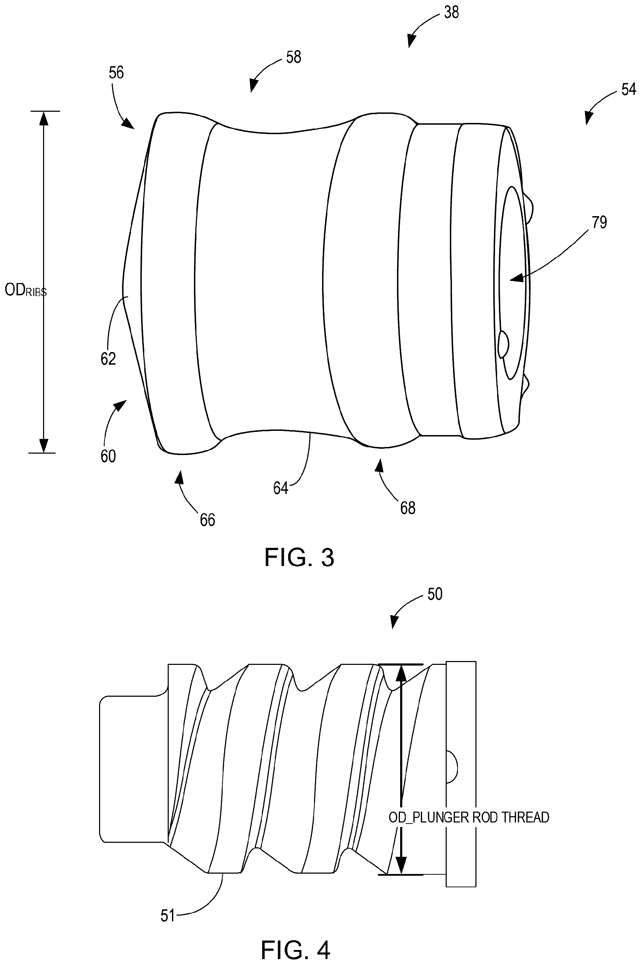

Referring now to FIGS. 3-7, and with continued reference to FIGS. 1 and 2, the structure of stopper 38 and extension member 50 of plunger rod 36 are shown in greater detail, according to an aspect or embodiment of the disclosure. The stopper 38 is defined by a main body 52 that includes an open proximal end 54 that engages with the distal end of plunger rod 36 and a closed distal end 56 configured to engage with the barrel 12 of syringe 10. The closed distal end 56 of the main body 52 includes a generally cylindrical portion 58 and a roof portion 60 that extends distally from cylindrical portion 58. In one embodiment, the roof portion 60 is configured as a conical portion that extends distally from cylindrical portion 58 to a tip 62 to provide the closed distal end 56. However, it is recognized that roof portion 60 may have other suitable shapes, such as being formed as a flat surface or domed surface, as other non-limiting examples.

An outer surface 64 of the main body 52 includes a plurality of ribs 66, 68 formed thereon that extend circumferentially around the entire outer surface 64. In the illustrated embodiment, the plurality of ribs 66, 68 includes a first rib 66 and a second rib 68, although it is recognized that the stopper 38 could include a greater number of ribs, such as three or four ribs, as explained further below. The first and second ribs 66, 68 are spaced apart longitudinally, such that the first rib 66 is positioned toward the proximal end 54 of the main body 52 and the second rib 68 is positioned toward the distal end 56 of the main body 52, with an interib region present between the first and second ribs 66, 68.

According to embodiments of the disclosure, the structure of each of first rib 66 and second rib 68 may be controlled to achieve a desired interaction between the stopper 38 and the barrel 12. According to one aspect of ribs 66, 68, the ribs 66, 68 extend radially outward a desired distance from the outer surface 64 (i.e., a rib thickness) to act as bearing points against the inner surface of the syringe barrel 12. In some embodiments, an outer diameter of the ribs, $OD_{ribs}$, is controlled based on an inner diameter of barrel 12, $ID_{barrel}$ (see FIG. 1), and to provide a desired amount of interference between the stopper 38 and the barrel 12. As one example, the rib thickness and the outer diameter of the ribs, $OD_{ribs}$, is such that an interference between the stopper 38 and the syringe barrel 12 is in the range of 2 to 10%, and preferably 4 to 10%, as determined by:

$$\text{Interference} = (OD_{ribs}/ID_{barrel}) - 1) \, x100.$$

According to aspects of the disclosure, the stopper 38 may further include one or more outer coatings or layers 70 of an inert material (see applied to portions thereof, such as on a sealant or gliding surface thereof - i.e., on a contact surface 72 of first rib 66 and second rib 68 - and/or on roof portion 60. The layer(s) 70 may be configured to reduce friction between the stopper 38 and barrel 12 when the stopper 38 is moved within the syringe 10. According to non-limiting embodiments, the layer(s) 70 may be formed of a fluoropolymer, an ultra-high-molecular-weight polyethylene (UHMWPE), or a thermoplastic elastomer (TPE) free of per- and poly- fluoroalkyl substances (PFAS).

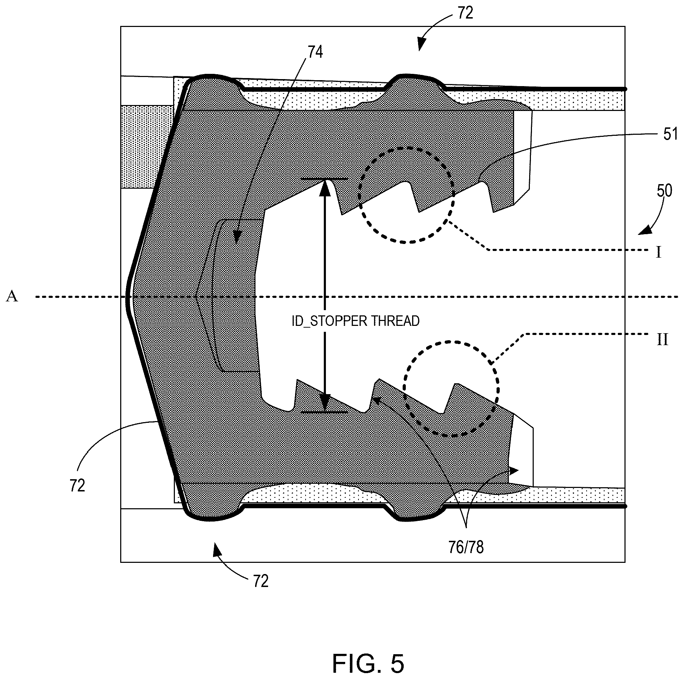

As shown in FIG. 5, the main body 52 of the stopper 38 is substantially hollow and is sized and configured to receive the extension member 50 of plunger rod 36 therein. The main body 52 of the stopper 38 defines an inner cavity 74 that is generally defined between a majority of the open proximal end 54 and the closed distal end 56 of the main body 52. With the inner cavity 74 formed as such, the second rib 68 is thus positioned at a location that is axially aligned (along longitudinal axis A) with a portion of the inner cavity 74. The inner cavity 74 includes a threaded inner surface 76 that is configured to receive and mate with the threaded extension member 50 of plunger rod 36. The threaded inner surface 76 includes a thread 78 therein that, according to embodiments, may be configured as a cylindrical thread or a conical thread, as non-limiting examples.

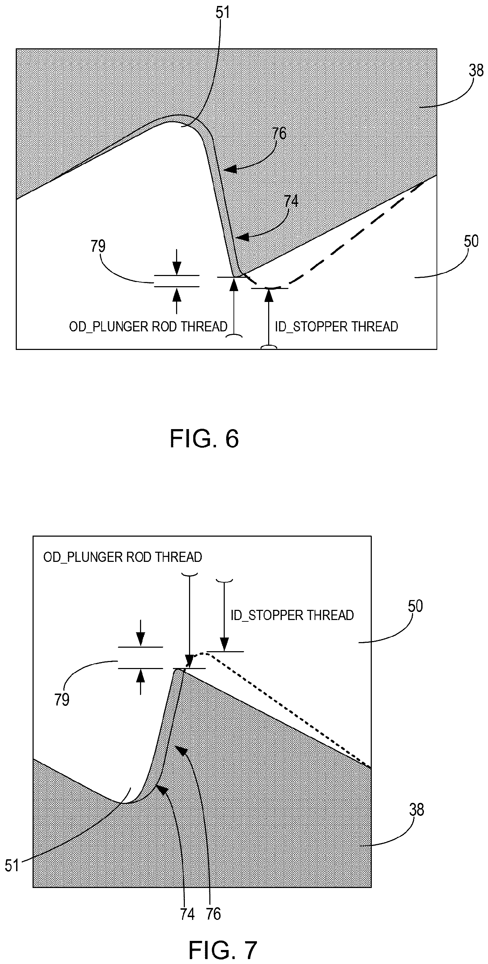

According to aspects of the disclosure, and as shown in FIGS. 5-7, the inner cavity 74 and the extension member 50 are configured such that an interference (indicated by "79" in FIGS. 6 and 7) is present between the threaded extension member 50 and the threaded inner cavity 74 when mated together. That is, the inner cavity 74 and the extension member 50 are configured such that the thread 51 of the extension member 50 is sized larger than the thread 78 of inner cavity 74. According to embodiments, the interference 79 between the threads 51, 78 of the threaded extension member 50 and the threaded inner cavity 74 may be determined by a pitch of the inner cavity thread 78 and a pitch of the extension member thread 51 and/or by an outer diameter of the extension member thread 51 and an inner diameter of the inner cavity thread 74. The interference 79 between the threads 51, 78 of the threaded extension member 50 and the threaded inner cavity 74 may be between 0.5% and 15%, as defined by:

$$\text{Interference} = (abs \, (ID\_stopper \, thread - OD\_plunger \, rod \, thread) \, / \, OD\_plunger \, rod \, thread)$$

where ID_stopper thread is an inner diameter of the stopper cavity thread and OD_plunger rod thread is an outer diameter of the plunger rod thread.

The interference 79 between the threads 51, 78 of the threaded extension member 50 and the threaded inner cavity 74 serves to apply a radially outward pressure compensation to the portion of the stopper 38 between the inner cavity 74 and the outer surface 64, with the radially outward pressure compensation thus also applied to the second rib 68 formed on this portion of the stopper 38 that is axially aligned with the threaded inner cavity 74. According to embodiments, the radially outward pressure compensation applied to the second rib 68 by the interference 79 between the threaded extension member 50 and the threaded inner cavity 74 is between 10 and 50% when measured as a correction ratio of the added value of the interference that counterbalances a contact pressure lost due to an empty thread, with it recognized that the exact value will depend on design space variation and on rib geometry principals. Alternatively, the radially outward pressure compensation applied to the second rib 68 by the interference 79 between the threaded extension member 50 and the threaded inner cavity 74 may be expressed as a contact pressure value.

The radially outward pressure compensation applied to the second rib 68 aids in maintaining a consistent contact pressure between the second rib 68 and the inner surface of syringe barrel 12, so as to help maintain CCI in the syringe 10. In some embodiments, a contact pressure of between 1 to 60MPA may be maintained between the second rib 68 and the inner surface of syringe barrel 12, which may be approximately equal to a contact pressure applied between the first rib 66 and the inner surface of syringe barrel 12.

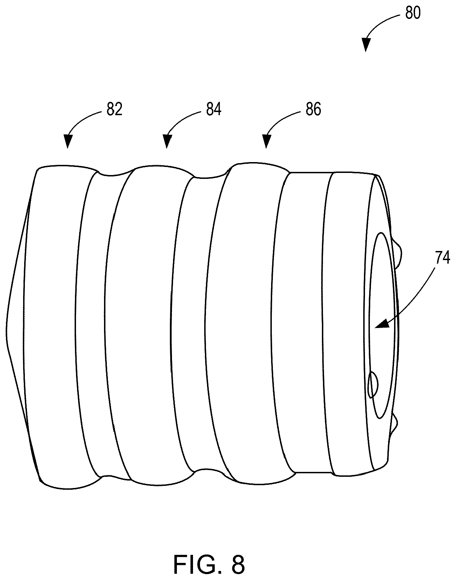

As previously indicated, while the stopper 38 is shown in FIGS. 3-5 as including only two (2) ribs 66, 68 formed thereon (i.e., first rib 66 and second rib 68), it is recognized that stopper 38 may be constructed to include a greater number of ribs formed thereon. FIG. 8 illustrates a stopper 80 having three (3) ribs thereon - a first rib 82, a second rib 84, and a third rib 86. Similar as to described above with stopper 38, stopper 80 may include an inner cavity 74 that mates with the extension member 50 of plunger rod 36 (FIGS. 1 and 2) - with the inner cavity 74 and extension member 50 configured such that an interference is present between the threaded extension member 50 and the threaded inner cavity 74 when mated together. That is, the inner cavity 74 and the extension member 50 are configured such that the thread of the extension member 50 is sized larger than the thread of inner cavity 74, so as to provide an interference there between of 0.5% and 15%, as a non-limiting example.

In the embodiment of FIG. 8, both the second rib 84 and the third rib 86 are positioned at a location that is axially aligned (along longitudinal axis A) with a portion of the inner cavity 74. Accordingly, the interference between the threads 51 of the threaded extension member 50 and the threaded inner cavity 74 serves to apply a radially outward pressure compensation to the portion of the stopper 80 between the cavity 74 and the outer surface 66, with the radially outward pressure compensation thus also applied to the second and third ribs 84, 86 that are axially aligned with portions of the threaded inner cavity 74. According to embodiments, the radially outward pressure compensation applied to the second and third ribs 84, 86 by the interference between the threaded extension member 50 and the threaded inner cavity 74 is between 10 and 50%. This radially outward pressure compensation applied to the second and third ribs 84, 86 in maintaining a consistent contact pressure between the second and third ribs 84, 86 and the inner surface of syringe barrel 12, so as to help maintain CCI in the syringe 10. In some embodiments, this radially outward pressure compensation may equate to a contact pressure of between 1 and 60MPA that may be maintained between the second and third ribs 84, 86 and the inner surface of syringe barrel 12.

Beneficially, embodiments of the invention thus are directed to syringe having a plunger assembly designed to maintain a consistent radial contact pressure between the stopper thereof and a syringe barrel. The stopper includes a threaded inner cavity that mates with a threaded distal extension member of a plunger rod of the plunger assembly, with the threaded inner cavity and threaded extension member configured such that an interference fit is formed therebetween when mated together. The interference fit causes a radially outward pressure compensation to be applied to one or more ribs of the stopper that are aligned with the inner cavity, with the radially outward pressure compensation aiding the ribs in maintaining a consistent contact pressure against the syringe barrel, so as to maintain contact enclosure integrity in the syringe.

Although the present disclosure has been described in detail for the purpose of illustration based on what is currently considered to be the most practical and preferred embodiments or aspects, it is to be understood that such detail is solely for that purpose and that the present disclosure is not limited to the disclosed embodiments or aspects, but, on the contrary, is intended to cover modifications and equivalent arrangements that are within the spirit and scope of the appended claims. For example, it is to be understood that the present disclosure contemplates that, to the extent possible, one or more features of any embodiment may be combined with one or more features of any other embodiment.

**Claims**

1.   A syringe comprising:

a syringe barrel having a barrel proximal end and a barrel distal end and defining a chamber configured for containing a fluid therein; and
a plunger assembly axially movable within the chamber of the syringe barrel between a retracted position and an advanced position, the plunger assembly including:

a plunger having a plunger rod proximal end and a plunger rod distal end, with a threaded extension member formed at the plunger rod distal end; and
a stopper secured to the plunger rod distal end, the stopper further including:

a main body portion defining a proximal end and a distal end, with the main body defining a threaded inner cavity at the proximal end configured to mate with the threaded extension member to secure the stopper to the plunger rod; and
a plurality of ribs extending from an outer surface of the main body portion and around an outer circumference of the main body portion, with at least one of the plurality of ribs positioned on the main body portion so as to axially aligned with a portion of the threaded inner cavity;

wherein with the threaded extension member mated with the threaded inner cavity, an interference is present between the threaded extension member and the threaded inner cavity that applies a radially outward pressure compensation to the at least one rib that is axially aligned with the threaded inner cavity.

2. The syringe of claim 1, wherein the plurality of ribs comprises a first rib and a second rib, and wherein the second rib is axially aligned with the portion of the threaded inner cavity.

3. The syringe of claim 2, wherein the plurality of ribs further comprises a third rib, and wherein each of the second rib and the third rib are axially aligned with portions of the threaded inner cavity.

4. The syringe of any of claims 1-3, wherein each of the threaded extension member and the threaded inner cavity comprises a thread, the thread configured as a cylindrical thread or a conical thread.

5. The syringe of claim 4, wherein the interference between the threads of the threaded extension member and the threaded inner cavity is between 0.5% and 15%, as defined by:

$$\text{Interference} = (\text{abs}\,(\text{ID\_stopper thread} - \text{OD\_plunger rod thread})\,/\,\text{OD\_plunger rod thread})$$

where ID_stopper thread is an inner diameter of the stopper cavity thread and OD_plunger rod thread is an outer diameter of the plunger rod thread.

6. The syringe of claim 5, wherein the interference is determined by a pitch of the inner cavity thread and a pitch of the extension member thread.

7. The syringe of claim 5, wherein the interference is determined by an outer diameter of the extension member thread and an inner diameter of the inner cavity thread.

8. The syringe of claim 7, wherein the thread of each of the threaded extension member and the threaded inner cavity comprises a crest, and wherein the crest of the threaded extension member or the threaded inner cavity is in interference with the other of the threaded extension member or the threaded inner cavity.

9. The syringe of any of claims 1-8, wherein the radially outward pressure compensation applied to the at least one rib by the interference between the threaded extension member and the threaded inner cavity is from 10 to 50%.

10. The syringe of any of claims 1-9, wherein another interference is present between the plurality of ribs and an inner surface of the syringe barrel.

11. The syringe of claim 10, wherein theanother interference between the plurality of ribs and the inner surface of the syringe barrel is from 2 to 10%, as determined by:

$$\text{Interference} = ((\text{OD}_{ribs}\,/\text{ID}_{barrel}) - 1)\,\text{x}\,100,$$

wherein $\text{OD}_{ribs}$ is an outer diameter of the ribs and $\text{ID}_{barrel}$ is an inner diameter of the syringe barrel.

12. The syringe of any of claims 1-11, wherein the plurality of ribs apply a contact pressure against the inner surface of the syringe barrel of between 1 and 60MPa.

13. The syringe of any of claims 1-12, wherein the stopper comprises a lubricant-free or low lubricant stopper.

14. The syringe of any of claims 1-12, wherein the stopper comprises one or more layers of inert material formed on the plurality of ribs and the outer surface of the main body portion.

15. The syringe of claim 14, wherein the inert material formed comprises one or more of a fluoropolymer, an ultra-high-molecular-weight polyethylene (UHMWPE), or a thermoplastic elastomer (TPE) free of per- and poly- fluoroalkyl substances (PFAS).

FIG. 1

**FIG. 2**

EP 4 613 300 A1

$OD_{RIBS}$

56  58  38  54  79  62  60  66  64  68

**FIG. 3**

50

OD_PLUNGER ROD THREAD

51

**FIG. 4**

72

74

51

50

I

A

ID_STOPPER THREAD

II

72

76/78

72

FIG. 5

FIG. 6

FIG. 7

FIG. 8

Europäisches
Patentamt

European
Patent Office

Office européen
des brevets

## EUROPEAN SEARCH REPORT

Application Number

EP 24 16 2403

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2005/010175 A1 (BEEDON DANIEL E [US] ET AL) 13 January 2005 (2005-01-13) * paragraphs [0058], [0066], [0067], [0071]; figures 1-6 * | 1-15 | INV. A61M5/315 |
| X | AU 2015 315 849 A1 (SIO2 MEDICAL PRODUCTS INC) 23 March 2017 (2017-03-23) * paragraphs [00155], [00234]; figure 8 * | 1-15 | |
| A | US 10 835 681 B2 (BECTON DICKINSON CO [US]) 17 November 2020 (2020-11-17) * column 2, lines 8-18 * | 1-15 | |

TECHNICAL FIELDS
SEARCHED       (IPC)

A61M

The present search report has been drawn up for all claims

| Place of search | Date of completion of the search | Examiner |
|---|---|---|
| Munich | 13 June 2024 | Fisentzou, Vikentios |

EPO FORM 1503 03.82 (P04C01)

## ANNEX TO THE EUROPEAN SEARCH REPORT
## ON EUROPEAN PATENT APPLICATION NO.

EP 24 16 2403

13-06-2024

| Patent document cited in search report | Publication date | Patent family member(s) | Publication date |
|---|---|---|---|
| US 2005010175 A1 | 13-01-2005 | EP 1596909 A2 | 23-11-2005 |
| | | JP 2006519070 A | 24-08-2006 |
| | | US 2005010175 A1 | 13-01-2005 |
| | | WO 2004075958 A2 | 10-09-2004 |
| AU 2015315849 A1 | 23-03-2017 | AU 2015315849 A1 | 23-03-2017 |
| | | AU 2020201401 A1 | 12-03-2020 |
| | | CA 2960135 A1 | 17-03-2016 |
| | | CN 107106782 A | 29-08-2017 |
| | | CN 112451808 A | 09-03-2021 |
| | | EP 3191159 A1 | 19-07-2017 |
| | | EP 3785751 A1 | 03-03-2021 |
| | | JP 6826028 B2 | 03-02-2021 |
| | | JP 2017526465 A | 14-09-2017 |
| | | KR 20170051488 A | 11-05-2017 |
| | | MY 178361 A | 09-10-2020 |
| | | NZ 729991 A | 28-02-2020 |
| | | NZ 759333 A | 26-11-2021 |
| | | RU 2017108403 A | 10-10-2018 |
| | | SG 10201903122S A | 30-05-2019 |
| | | SG 11201701640T A | 30-03-2017 |
| | | US 2017296756 A1 | 19-10-2017 |
| | | US 2021077742 A1 | 18-03-2021 |
| | | WO 2016039816 A1 | 17-03-2016 |
| US 10835681 B2 | 17-11-2020 | US 2018056006 A1 | 01-03-2018 |
| | | WO 2016153912 A1 | 29-09-2016 |